# FASCICULE DE BREVET EUROPEEN

(11) **EP 0 745 370 B1**
(45) Date de publication et mention de la délivrance du brevet: **28.03.2001**
(21) Numéro de dépôt: 96400834.6
(22) Date de dépôt: 18.04.1996
(51) Int. Cl.: A61K 7/02, A61K 7/48

(54) **Composition sous forme de gel anhydre à phase grasse sans cire, contenant une argile organomodifiée, des particules creuses thermoplastiques expansées et une silice pyrogénée et utilisations en application topique**
Zusammensetzung in Form eines wasserfreien Gels mit wachsfreier Fettphase, enthaltend organomodifizierten Ton, expandierte hohle thermoplastische Partikel und pyrogene Kieselsäure zur topischen Anwendung
Composition in the form of an anhydrous gel with wax-free fatty phase containing an organomodified clay, expanded hollow thermoplastic particles and a pyrogenic silica and its use in topical application

(30) Priorité: 30.05.1995 FR 9506387
(43) Date de publication de la demande: 04.12.1996
(73) Titulaire: L'OREAL, 75008 Paris (FR)
(72) Inventeur: Perrin, Martine, 91600 Savigny sur Orge (FR); Terren, Nadia, 94550 Chevilly Larue (FR); Michelet, Jacques, 91160 Champlan (FR)
(74) Mandataire: Dodin, Catherine

(56) Documents cités:
- EP-A- 0 605 284
- FR-A- 2 486 800
- GB-A- 1 372 701
- US-A- 4 431 673
- US-A- 5 266 321

## Description

La présente invention a trait à une composition sous forme de gel anhydre à phase grasse sans cire et contenant une argile organomodifiée, des particules creuses thermoplastiques expansées et de la silice pyrogénée ainsi que ses utilisations en application topique notamment en cosmétique et/ou en dermatologie.

Les compositions de maquillage contenant une phase grasse sont couramment utilisées en cosmétique en raison de leur bonne adhérence à l'épiderme, leur sensation de confort, leur capacité protectrice et leur capacité à former un film imperméable à l'eau. Les produits anhydres de maquillage se présentent en général sous forme solide compacte ou bien sous forme de crème.
Les compositions ayant une consistance onctueuse, sont plus recherchées dans la mesure où elles peuvent être appliquées de façon satisfaisante à l'aide des doigts à partir d'un conditionnement sous forme de pot ou de tube. Elles contiennent généralement des cires afin d'obtenir une consistance stable dans le temps.
Ces produits à base de cires sont obtenus par fusion des cires à des températures élevées, généralement supérieures à 80°C, de manière à pouvoir les intégrer de manière homogène dans leur formulation. Il s'avère impossible d'incorporer dans ce type de compositions des actifs cosmétiques ou dermatologiques thermosensibles.
On cherche donc à réaliser des produits de maquillage gras sans cire pouvant être fabriqués à température ambiante de façon à pouvoir incorporer des actifs thermosensibles et dont la texture soit suffisamment onctueuse pour permettre un étalement satisfaisant avec les doigts. Cependant en l'absence de cire, on pourrait s'attendre à un manque de stabilité dans le temps de ces produits, à des phénomènes de ramollissement ou de relargage d'huile entraînant une texture grasse et collante ainsi qu'un étalement difficile.

La Demanderesse a découvert de manière surprenante que l'utilisation de l'association d'une argile organomodifiée, de particules creuses thermoplastiques de polymère ou copolymère expansé formé à partir d'un monomère ou d'un mélange de monomères à insaturation éthylénique et de silice pyrogénée, dispersée dans une phase grasse ne contenant pas de cire, conduisait à des compositions sous forme de gels anhydres, stables dans le temps et présentant une texture homogène, non grasse, douce, non collante, fondante et suffisamment onctueuse pour être étalée de façon satisfaisante avec les doigts.
Ces compositions peuvent être préparées par simple mélange et homogénéisation à température ambiante et peuvent ainsi contenir des actifs thermosensibles.

Les compositions conformes à l'invention sont caractérisées par le fait qu'elle comprennent dans une phase grasse ne contenant pas de cire au moins :
a) une argile organomodifiée ;
b) des particules creuses thermoplastiques expansées de polymère ou de copolymère formé à partir d'un monomère ou d'un mélange de monomères à insaturation éthylénique ; et
c) une silice pyrogénée.

La phase grasse constituant la base des compositions de l'invention représente, de préférence, de 60 à 85 % en poids et plus particulièrement de 65 à 77 % en poids du poids total de la composition.

La phase grasse est constituée d'au moins une huile choisie parmi :
- les huiles d'origine minérale, végétale ou animale telles que l'huile de vaseline, l'huile de soja, l'huile de tournesol, l'huile de sésame, l'huile de colza, l'huile d'amande douce, l'huile de macadamia, l'huile de pépins de cassis, le beurre de karité et sa fraction liquide ou le perhydrosqualène ;
- les huiles de synthèse telles que les esters d'acides gras ou les alcools gras comme par exemple : le parléam, le néopentanoate d'isostéaryle, le myristate d'isopropyle, le stéarate d'isopropyle, le palmitate d'éthyl-2 hexyle, le lanolate d'isopropyle, l'isononanoate d'isononyle, l'isononanoate d'isotridécyle, le palmitate d'octyle, l'alcool oléique, les triglycérides d'acides gras saturés d'origine végétale tels que les triglycérides des acides caprylique/caprique comme ceux vendus par la société STEARINERIES DUBOIS ou ceux vendus sous les dénominations MIGLYOL 810, 812 et 818 par la société DYNAMIT NOBEL, le polyisobutène hydrogéné ;
- les huiles siliconées telles que les cyclométhicones comme par exemple le cyclopentadiméthylsiloxane et le cyclohexadiméthylsiloxane ; les polydiméthylsiloxanes;
- les gommes de silicone phénylées ou hydroxylées ;
- les huiles fluorées ;
- leurs mélanges.

Les argiles organomodifiées sont de préférence choisies parmi les produits de réaction d'une hectorite ou d'une montmorillonite et d'un sel d'ammonium quaternaire en particulier le chlorure de stéaryldiméthylbenzylammonium ou le chlorure d'ammonium quaternaire de formule : 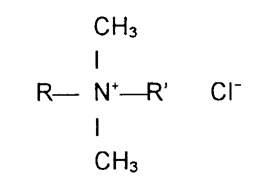 dans laquelle R et R' représentent un mélange de restes d'alkyle ayant de 14 à 20 atomes de carbone dérivés de suif hydrogéné.

Selon l'invention, les argiles organomodifiées sont présentes à des concentrations allant préférentiellement de 0,5 à 6% en poids en matière active et plus particulièrement de 2 à 5% en poids par rapport au poids total de la composition.

On utilise plus particulièrement les hectorites ou montmorillonites organomodifiées sous forme de gels constitués de ladite argile organomodifiée, d'une huile d'origine minérale, végétale ou animale ou synthétique telles que celles définies ci-dessus et d'un agent mouillant tel que le carbonate de propylène.
A titre d'exemple, on peut citer les produits vendus sous les dénominations MlGLYOL GEL B, MIGLYOL GEL T, MIGLYOL 840 GEL B, MIGLYOL 840 GEL T par la société DYNAMIT NOBEL. Ces produits comportent une hectorite ou une montmorillonite modifiée par un sel de chlorure de stéaryldiméthylbenzylammonium, un mélange de triglycérides des acides caprique et caprylique et du carbonate de propylène.

Les particules utilisables selon l'invention peuvent être réalisées à partir de monomères à insaturation éthylénique, non toxiques et non irritants pour la peau.
Les particules de l'invention peuvent être obtenues, par exemple, selon les procédés des brevets et demandes de brevet EP-056219, EP-348372, EP-486080, EP-320473, EP-112807 et US-3615972.
La cavité interne des particules contient en principe un gaz qui peut être de l'air, de l'azote ou un hydrocarbure comme l'isobutane ou l'isopentane.
Parmi les monomères utilisés pour la réalisation des particules creuses thermoplastiques expansées de l'invention, on peut citer les esters d'acide méthacrylique ou acrylique tels que l'acrylate ou le méthacrylate de méthyle ; le chlorure de vinylidène ; l'acrylonitrile ; le styrène et ses dérivés ; le butadiène et ses dérivés ; leurs mélanges.
On peut par exemple utiliser les polymères ou copolymères d'acrylate ou méthacrylate de méthyle, les copolymères formés à partir de styrène et d'acrylonitrile, les copolymères de chlorure de vinylidène et d'acrylonitrile ou de chlorure de vinyle.
On utilise de préférence un polymère ou copolymère contenant : de 0% à 60% de chlorure de vinylidène ou d'un de ses dérivés, de 20% à 90% d'acrylonitrile ou d'un de ses dérivés et de 0% à 50% d'un monomère (méth)acrylique ou styrènique, la somme des pourcentages (en poids) étant égale à 100. le monomère (méth)acrylique est par exemple le (méth)acrylate de méthyle ou d'éthyle. Le monomère styrènique est par exemple le styrène ou le α-méthyl-styrène.
Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.

De façon avantageuse, les particules de l'invention ont une granulométrie allant de 1 µm à 100 µm et encore mieux allant de 5 µm à 60 µm, et encore mieux de 10 µm à 50 µm.
De façon préférentielle, la masse volumique des particules est choisie dans la gamme allant de 15 kg/m³ à 200 kg/m³ et mieux de 40 kg/m³ à 120 kg/m³, et encore mieux de 60 kg/m³ à 80 kg/m³.
Les particules utilisables dans l'invention sont par exemple les microsphères de terpolymère expansé de chlorure de vinylidène, d'acrylonitrile et de méthacrylate, vendues sous la marque EXPANCEL par la société Nobel Casco sous les références 551 DE 50 (granulométrie d'environ 40 µm), 551 DE 20 (granulométrie d'environ 30µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23.
Les particules creuses thermoplastiques expansées conformes à l'invention sont présentes dans des concentrations allant préférentiellement de 0,1 à 3% en poids et plus particulièrement de 0,5 à 2% en poids par rapport au poids de la composition.

La silice pyrogénée présente dans les compositions de l'invention peut être sous forme de silice pyrogénée hydrophile ou hydrophobe. Les silices pyrogénées sont obtenues par hydrolyse à haute température d'un composé volatil de silicium dans une flamme oxhydrique. Il s'ensuit la production d'une silice finement divisée. Par réaction, on peut modifier chimiquement la surface par réduction de groupes silanols afin d'obtenir une silice hydrophobe par exemple avec un diméthylsiloxane ou un triméthylsiloxane.
On utilise en particulier les produits vendus par les dénominations AEROSILS 200, R 972 et R 974 par la société DEGUSSA.
La silice pyrogénée est, de préférence, présente dans les compositions de l'invention à des concentrations allant de 0,1 à 3% en poids par rapport au poids total de la composition.

Une forme particulièrement préférée de composition selon l'invention contient en plus un gel de silicone constitué d'un organopolysiloxane partiellement réticulé de structure tridimensionnelle inclus dans une huile de silicone de faible viscosité. L'ajout de ce gel siliconé permet d'améliorer les qualités de douceur et de fondant des compositions de l'invention.

Les gels de silicone utilisés selon l'invention sont caractérisés par le fait qu'ils sont constitués de :
A) un polyorganosiloxane de structure tridimensionnelle partiellement réticulé choisi parmi:
   i) les polyorganopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
   ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non cyclique et entre 1 et 50% mol lorsque l'organopolysiloxane est cyclique ;
B) une huile de silicone de viscosité inférieure ou égale à 0,5 Pa.s ; le rapport en poids polyorganosiloxane partiellement réticulé/huile de silicone étant compris entre 5/95 et 30/70.

Les huiles de silicone de faible viscosité sont choisies, par exemple, parmi un diméthylpolysiloxane ou un méthylphénylpolysiloxane linéaire à faible degré de polymérisation, un octaméthylcyclopentasiloxane, un décaméthylcyclopentasiloxane ou un mélange de ces deux produits.

Les gels de silicone utilisés selon l'invention sont connus et sont décrits dans le brevet US 5.266.321. IIs sont préparés selon les procédés définis dans ce même document.
On utilise en particulier le produit vendu sous la dénomination KSG16 par la société SHIN ETSU. Ce produit contient un mélange constitué d'un polydiméthylsiloxane linéaire de faible viscosité (0,06 Pa.s) et d'un polydiméthylsiloxane partiellement réticulé de structure tridimensionnelle.
Les polyorganosiloxanes partiellement réticulés sont présents dans les compositions de l'invention à des concentrations en matière active allant de préférence jusqu'à environ 8% en poids par rapport au poids total de la composition.

Les compositions selon l'invention peuvent se présenter sous forme de fonds de teints ou de crèmes teintées et contenir des pigments minéraux ou organiques.

Parmi les pigments organiques, on peut citer les laques telles que les laques de calcium de D et C Red n°7, les laques de baryum des D et C Red n°6 et 9, les laques d'aluminium des D et C Red n°3 et de D et C Yellow n°5 et les laques de zirconium de D et C Orange n°5. Parmi les pigments minéraux, on peut citer : les oxydes de fer (rouge, brun, noir et jaune), les oxydes de chrome, les outremers (polysulfures d'aluminosilicates), le dioxyde de titane, le pyrophosphate de manganèse et le bleu de Prusse (ferrocyanure ferrique).

Les pigments sont présents dans des concentrations allant préférentiellement de 0 à 20% en poids et plus particulièrement de 3 à 12% en poids par rapport au poids total de la composition.

Les compositions selon l'invention peuvent contenir également des agents matifiants tels que par exemple le carbonate de magnésium, de l'amidon ou des amidons modifiés, de la poudre de polyéthylène, de la poudre de zinc, de l'oxyde de zinc, du stéarate de magnésium ou de zinc, des microbilles de résine siliconée telles que le produit vendu sous le nom TOSPEARL par la société TOSHIBA, des microsphères de silice. Ils sont présents dans des concentrations allant préférentiellement de 2 à 15% en poids et plus particulièrement de 5 à 10% en poids par rapport au poids total de la composition.

Les compositions selon l'invention peuvent également contenir des actifs cosmétiques ou dermatologiques tels que la vitamine A, la vitamine E, le palmitate de vitamine A et la vitamine F et des additifs tels que des conservateurs, des parfums, des filtres solaires, des antioxydants, des bactéricides, des hydratants, de la mélanine, des charges telles que le talc, le kaolin, le mica, les nanotitanes.

Un autre objet de l'invention consiste en des compositions cosmétiques et/ou dermatologiques caractérisées par le fait qu'elles sont constituées des compositions telles que définies ci-dessus.

Les compositions selon l'invention peuvent être utilisées comme bases de produits de maquillage tels que des fonds de teint, des embelliseurs de teint, des correcteurs de maquillage, des crèmes teintées, des fards à paupières ou bien comme bases de produits pour le soin de la peau et /ou du visage tels que, par exemple, des crèmes blanches pour le lissage du relief autour des yeux.
Elles sont de préférence conditionnées dans des tubes ou pots et sont appliquées facilement avec les doigts sur la peau et/ou le visage.

Bien entendu, l'homme de l'art veillera à choisir les éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Les exemples qui suivent servent à illustrer l'invention sans toutefois présenter un caractère limitatif.

### Exemple 1: Fond de teint

- Hectorite modifiée par le chlorure de stéaryldiméthyl- ammonium sous forme de gel (MIGLYOL GEL B de DYNAMIT NOBEL) 30,0%
- Particules expansées de copolymère de chlorure de vinylidène/acrylonitrile/méthacrylate enfermant de l'isobutane (EXPANCEL 550 DE 20 de NOBEL CASCO) 1,5%
- Silice pyrogénée hydrophobe modifiée en surface par le triméthylsiloxane (AEROSIL R 972 de DEGUSSA) 1,0%
- Gel de silicone constitué d'un mélange de polydiméthylsiloxane (0,06 Pa.s)/polydiméthylsiloxane réticulé (75/25) vendu sous le nom KSG16 par SHIN ETSU 15,0%
- Pigments 12,0%
- Filtre solaire 4,0%
- Talc 8,0%
- Parfum 0,3%
- Cyclopentadiméthylsiloxane 19,5%
- Néopentanoate d'isostéaryle 8,7%
La composition est préparée de la manière suivante :
On opère à température ambiante. On disperse les pigments dans l'huile de silicone volatile. On ajoute le gel à base d'hectorite modifiée et le gel de silicone sous agitation. On ajoute la silice pyrogénée et on homogénéise l'ensemble. On incorpore ensuite le parfum, les actifs, les particules expansées creuses, au préalable empâtés dans le néopentanoate d'isostéaryle. On introduit ensuite le talc. La dispersion est affinée en final par broyage.

### Exemple 2: Embellisseur de teint

- Hectorite modifiée par le chlorure de stéaryldiméthylammonium sous forme de gel vendu sous le nom MIGLYOL GEL B par DYNAMIT NOBEL 38,0%
- Particules expansées de copolymère de chlorure de vinylidène/acrylonitrile/méthacrylate enfermant de l'isobutane (EXPANCEL 550 DE 20 de NOBEL CASCO) 1,5%
- Silice pyrogénée hydrophobe modifiée en surface par le triméthylsiloxane (AEROSIL R 972 de DEGUSSA) 1,0%
- Gel de silicone constitué d'un mélange de polydiméthylsiloxane (0,06 Pa.s)/polydiméthylsiloxane réticulé (75/25) vendu sous le nom KSG16 par SHIN ETSU 12,0%
- Pigments 4,5%
- Talc 11,0%
- Parfum 0,3%
- D,L-alpha-tocophérol (actif) 0,1%
- Cyclopentadiméthylsiloxane 21,6%
- Cyclohexadiméthylsiloxane 2,0%
- Néopentanoate d'isostéaryle 8,0%
La formulation de l'exemple 2 est préparée selon le même procédé que celui de l'exemple 1.

### Exemple 3: Embellisseur abricot

- Hectorite modifiée par le chlorure de stéaryldiméthylammonium sous forme de gel vendu sous le nom MIGLYOL GEL B par DYNAMIT NOBEL 35,0%
- Particules expansées de copolymère de chlorure de vinylidène/acrylonitrile/méthacrylate enfermant de l'isobutane (EXPANCEL 550 DE 20 de NOBEL CASCO) 1,5%
- Silice pyrogénée hydrophobe modifiée en surface par le triméthylsiloxane (AEROSIL R 972 de DEGUSSA) 1,0%
- Gel de silicone constitué d'un mélange de polydiméthylsiloxane (0,06 Pa.s)/polydiméthylsiloxane réticulé (75/25) vendu sous le nom KSG16 par SHIN ETSU 15,0%
- Pigments 4,5%
- Talc 8,0%
- Parfum 0,3%
- D,L-alpha-tocophérol (actif) 0,2%
- Cyclopentadiméthylsiloxane 24,5%
- Néopentanoate d'isostéaryle 10,0%
La formulation de l'exemple 3 est préparée selon le même procédé que celui de l'exemple 1.

### Exemple 4 : Correcteur de maquillage

- Hectorite modifiée par le chlorure de stéaryldiméthylammonium sous forme de gel vendu sous le nom MIGLYOL GEL B par DYNAMIT NOBEL 40,0%
- Particules expansées de copolymère de chlorure de vinylidène/acrylonitrile/méthacrylate enfermant de l'isobutane (EXPANCEL 550 DE 20 de NOBEL CASCO) 0,5%
- Silice pyrogénée hydrophile (AEROSIL 200 de DEGUSSA) 1,0%
- Parahydroxybenzoate de propyle 0,2%
- Pigments 5,0%
- Mica 3,0%
- Amidon réticulé par l'épichlorhydrine 4,0%
- Talc 3,0%
- Parfum 0,2%
- Triglycérides d'acides caprylique/caprique(60/40) 14,1%
- Cyclopentadiméthylsiloxane 29,0%
La composition est préparée de la manière suivante :
On opère à température ambiante. On disperse les pigments dans l'huile de silicone volatile. On ajoute le gel à base d'hectorite modifiée, sous agitation. On ajoute la silice pyrogénée et on homogénéise l'ensemble. On incorpore ensuite le parfum, les actifs, les particules expansées creuses, au préalable empâtés dans les triglycérides d'acides caprylique/caprique. On introduit ensuite le talc et l'amidon réticulé. La dispersion est affinée en final par broyage.

Les compositions des exemples 1 à 4 sont stables à la centrifugation après 1 heure à une accélération de 900G et au stockage après 2 mois à température ambiante, à 37 et 45°C. Elles restent lisses sans relargage huileux.

Elles présentent une texture onctueuse caractérisée par :
**(1) leur pénétrométrie** : avec une pénétration de 20 à 40 g mesurée à l'analyseur de texture STEVENS avec un mobile cône, se déplaçant à une vitesse de 0,5 mm/s et pour une profondeur de 5 mm.
**(2)leur viscosité** : elle se situe environ entre 10 et 21 Pa.s après 10 minutes d'agitation, mesurée avec un appareil CONTRAVE, mobile 4.

## Revendications

1. Composition sous forme de gel anhydre à phase grasse, caractérisée par le fait qu'elle comprend dans une phase grasse ne contenant pas de cire au moins :
a) une argile organomodifiée ;
b) des particules creuses thermoplastiques expansées de polymère ou de copolymère formé à partir d'un monomère ou d'un mélange de monomères à insaturation éthylénique; et
c) une silice pyrogénée.

2. Composition selon la revendication 1, caractérisée que la phase grasse représente de 60 à 85% en poids du poids total de la composition.

3. Composition selon l'une quelconque des revendications 1 et 2, caractérisée par le fait que la phase grasse comprend au moins une huile choisie parmi les huiles végétales, les huiles minérales, les huiles synthétiques, les huiles siliconées, les gommes de silicone phénylées ou hydroxylées, les huiles fluorées ou leurs mélanges.

4. Composition selon l'une quelconque des revendications 1 à 3, caractérisée par le fait que l'argile organomodifiée est un produit de réaction d'une hectorite ou d'une montmorillonite et d'un sel d'ammonium quaternaire.

5. Composition selon la revendication 4, caractérisée par le fait que le sel d'ammonium quaternaire est le chlorure de stéaryldiméthylbenzylammonium ou le chlorure d'ammonium quaternaire de formule : 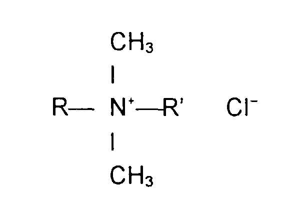 dans laquelle R et R' représentent un mélange de restes d'alkyle ayant de 14 à 20 atomes de carbone dérivés de suif hydrogéné.

6. Composition selon l'une quelconque des revendications 1 à 5, caractérisée par le fait que l'argile organomodifiée est présente dans des concentrations allant de 0,5 à 6 % en poids en matière active par rapport au poids total de la composition.

7. Composition selon l'une quelconque des revendications 1 à 6, caractérisée par le fait que l'argile organomodifiée est sous forme d'un gel constitué de ladite argile, d'une huile végétale, minérale, animale ou synthétique et d'un agent mouillant.

8. Composition selon la revendication 7, caractérisée par le fait que l'argile organomodifiée est sous forme d'un gel constitué de ladite argile, d'un mélange de triglycérides des acides caprique et caprylique et de carbonate de propylène.

9. Composition selon l'une quelconque des revendications 1 à 8, caractérisée par le fait que les particules creuses thermoplastiques expansées sont des particules de polymère ou de copolymère expansé formé à partir d'un monomère ou d'un mélange de monomères choisis dans le groupe formé par les esters d'acide méthacrylique ou acrylique; le chlorure de vinylidène ; l'acrylonitrile ; le styrène et ses dérivés ; le butadiène et ses dérivés.

10. Composition selon la revendication 9, caractérisée par le fait que les particules creuses thermoplastiques sont des particules de polymère ou copolymère d'acrylate ou méthacrylate de méthyle, de copolymère de styrène et d'acrylonitrile ou bien de copolymère de chlorure de vinylidène et d'acrylonitrile ou de chlorure de vinyle.

11. Composition selon la revendication 9 ou 10, caractérisée par le fait que le polymère ou copolymère contient de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100.

12. Composition selon la revendication 11, caractérisée par le fait que les particules creuses thermoplastiques expansées sont des particules creuses d'un copolymère expansé de chlorure de vinylidène et d'acrylonitrile ou un terpolymère expansé de chlorure de vinylidène, d'acrylonitrile et de méthacrylate de méthyle.

13. Composition selon l'une quelconque des revendications 9 à 12, caractérisée par le fait que les particules ont une granulométrie de 1 à 100 µm.

14. Composition selon l'une quelconque des revendications 9 à 13, caractérisée par le fait que les particules creuses thermoplastiques ont une masse volumique allant de 15 à 200 kg/m³.

15. Composition selon l'une quelconque des revendications 1 à 14, caractérisée par le fait que les particules creuses thermoplastiques expansées sont présentes dans des concentrations allant de 0,1 à 3% en poids par rapport au poids total de la composition.

16. Composition selon l'une quelconque des revendications 1 à 15, caractérisée par le fait que la silice pyrogénée est hydrophile ou hydrophobe.

17. Composition selon l'une quelconque des revendications 1 à 16, caractérisée par le fait que la silice pyrogénée est présente dans des concentrations allant de 0,1 à 3% en poids par rapport au poids total de la composition.

18. Composition selon l'une quelconque des revendications 1 à 17, caractérisée par le fait qu'elle contient en plus un gel de silicone constitué de :
A) un polyorganosiloxane de structure tridimensionnelle partiellement réticulé choisi parmi :
i) les polyorganopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle, un aryle, un groupe aliphatique insaturé et où le rapport en poids des motifs R₂SiO sur les motifs RSiO_{1,5} varie de 1/1 à 30/1 ;
ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non cyclique et entre 1 et 50% mol lorsque l'organopolysiloxane est cyclique ;
B) une huile de silicone de viscosité inférieure ou égale à 0,5 Pa.s;
le rapport en poids polyorganosiloxane partiellement réticulé/huile de silicone étant compris entre 5/95 et 30/70.

19. Composition selon la revendication 18, caractérisée par le fait que l'huile de silicone est choisi parmi un diméthylpolysiloxane ou un méthylphénylpolysiloxane linéaire à faible degré de polymérisation, un octaméthylcyclopentasiloxane, un décaméthylcyclopentasiloxane ou un mélange de ces deux produits.

20. Composition selon la revendication 18 ou 19, caractérisé par le fait que le gel de silicone est constituée d'un polydiméthylsiloxane linéaire de faible viscosité et d'un polydiméthylsiloxane partiellement réticulé de structure tridimensionnelle.

21. Composition selon l'une quelconque des revendications 18 à 20, caractérisée par le fait que le polyorganosiloxane partiellement réticulé est présent dans des concentrations allant jusqu'à environ 8% en poids en matière active par rapport au poids total de la composition.

22. Composition selon l'une quelconque des revendications 1 à 21, caractérisée par le fait qu'elle contient en plus au moins un pigment et/ou au moins un agent matifiant.

23. Composition selon la revendication 22, caractérisée par le fait qu'elle contient jusqu'à 20% en poids de pigment et/ou de 2 à 15% en poids d'agent matifiant.

24. Composition selon l'une quelconque des revendications 1 à 23, caractérisée par le fait qu'elle contient en plus des adjuvants choisis parmi des actifs cosmétiques ou dermatologiques, des antioxydants, les bactéricides, la mélanine, des hydratants, des filtres solaires, des parfums, des conservateurs, des charges.

25. Composition cosmétique et/ou dermatologique, caractérisée par le fait qu'elle est constituée par une composition telle que définie selon l'une quelconque des revendications 1 à 21.

26. Utilisation d'une composition telle que définie selon l'une quelconque des revendications 1 à 25 comme base de produits de maquillage ou de produits pour le soin de la peau et /ou du visage.

27. Utilisation selon la revendication 26, selon laquelle les produits sont conditionnés en tube ou en pot et sont destinés à être directement appliqués avec les doigts sur la peau et/ou le visage.

## Claims

1. Composition in the form of an anhydrous gel with a fatty phase, characterized in that it comprises, in a fatty phase containing no wax, at least:
a) an organomodified clay;
b) expanded thermoplastic hollow particles of polymer or of copolymer formed from a monomer or from a mixture of monomers containing ethylenic unsaturation; and
c) a pyrogenous silica.

2. Composition according to Claim 1, characterized in that the fatty phase represents from 60 to 85 % by weight of the total weight of the composition.

3. Composition according to either of Claims 1 and 2, characterized in that the fatty phase comprises at least one oil chosen from plant oils, mineral oils, synthetic oils, silicone oils, phenylated or hydroxylated silicone gums, fluoro oils or mixtures thereof.

4. Composition according to any one of Claims 1 to 3, characterized in that the organomodified clay is a product of reaction of a hectorite or a montmorillonite and a quaternary ammonium salt.

5. Composition according to Claim 4, characterized in that the quaternary ammonium salt is stearyldimethylbenzylammonium chloride or the quaternary ammonium chloride of formula: 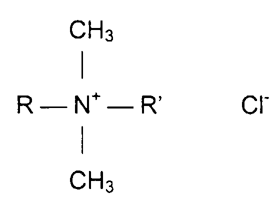 in which R and R' represent a mixture of alkyl residues having from 14 to 20 carbon atoms derived from hydrogenated tallow.

6. Composition according to any one of Claims 1 to 5, characterized in that the organomodified clay is present in concentrations ranging from 0.5 to 6 % by weight of active material relative to the total weight of the composition.

7. Composition according to any one of Claims 1 to 6, characterized in that the organomodified clay is in the form of a gel consisting of the said clay, a plant, mineral, animal or synthetic oil and a wetting agent.

8. Composition according to Claim 7, characterized in that the organomodified clay is in the form of a gel consisting of the said clay, a mixture of capric and caprylic acid triglycerides and propylene carbonate.

9. Composition according to any one of Claims 1 to 8, characterized in that the expanded thermoplastic hollow particles are particles of expanded polymer or copolymer formed from a monomer or a mixture of monomers chosen from the group formed by methacrylic or acrylic acid esters; vinylidene chloride; acrylonitrile; styrene and derivatives thereof; butadiene and derivatives thereof.

10. Composition according to Claim 9, characterized in that the thermoplastic hollow particles are particles of methyl acrylate or methacrylate polymer or copolymer, of copolymer of styrene and acrylonitrile or alternatively of copolymer of vinylidene chloride and acrylonitrile or vinyl chloride.

11. Composition according to Claim 9 or 10, characterized in that the polymer or copolymer contains from 0 % to 60 % of units derived from vinylidene chloride, from 20 % to 90 % of units derived from acrylonitrile and from 0 % to 50 % of units derived from an acrylic or styrene monomer, the sum of the percentages (by weight) being equal to 100.

12. Composition according to Claim 11, characterized in that the expanded thermoplastic hollow particles are hollow particles of an expanded copolymer of vinylidene chloride and acrylonitrile or an expanded terpolymer of vinylidene chloride, acrylonitrile and methyl methacrylate.

13. Composition according to any one of Claims 9 to 12, characterized in that the particles have a particle size of from 1 to 100 µm.

14. Composition according to any one of Claims 9 to 13, characterized in that the thermoplastic hollow particles have a density ranging from 15 to 200 kg/m³.

15. Composition according to any one of Claims 1 to 14, characterized in that the expanded thermoplastic hollow particles are present in concentrations ranging from 0.1 to 3 % by weight relative to the total weight of the composition.

16. Composition according to any one of Claims 1 to 15, characterized in that the pyrogenous silica is hydrophilic or hydrophobic.

17. Composition according to any one of Claims 1 to 16, characterized in that the pyrogenous silica is present in concentrations ranging from 0.1 to 3 % by weight relative to the total weight of the composition.

18. Composition according to any one of Claims 1 to 17, characterized in that it also contains a silicone gel consisting of:
A) a partially crosslinked polyorganosiloxane of three-dimensional structure chosen from:
i) polyorganopolysiloxanes comprising units R2SiO and RSiO_{1.5} and optionally units R₃SiO_{0.5} and/or SiO₂ in which the radicals R, independently of each other, denote a hydrogen, an alkyl, an aryl and an unsaturated aliphatic group and in which the weight ratio of the units R₂SiO to the units RSiO_{1.5} ranges from 1/1 to 30/1;
ii) insoluble polyorganopolysiloxanes which are swellable in silicone oil, obtained by addition of an organohydrogenopolysiloxane (1) and an organopolysiloxane (2) having unsaturated aliphatic groups, such that the amount of hydrogen or of unsaturated aliphatic groups in (1) and (2) respectively is between 1 and 20 mol% when the organopolysiloxane is non-cyclic and between 1 and 50 mol% when the organopolysiloxane is cyclic;
B) a silicone oil of viscosity less than or equal to 0.5 Pa.s;
the partially crosslinked polyorganosiloxane/silicone oil weight ratio being between 5/95 and 30/70.

19. Composition according to Claim 18, characterized in that the silicone oil is chosen from a linear methylphenylpolysiloxane or dimethylpolysiloxane with a low degree of polymerization, an octamethyl-cyclopentasiloxane, a decamethylcyclopentasiloxane or a mixture of these two products.

20. Composition according to Claim 18 or 19, characterized in that the silicone gel consists of a linear polydimethylsiloxane of low viscosity and a partially crosslinked polydimethylsiloxane of three-dimensional structure.

21. Composition according to any one of Claims 18 to 20, characterized in that the partially crosslinked polyorganosiloxane is present in concentrations ranging up to about 8 % by weight of active material relative to the total weight of the composition.

22. Composition according to any one of Claims 1 to 21, characterized in that it also contains at least one pigment and/or at least one matt-effect agent.

23. Composition according to Claim 22, characterized in that it contains up to 20 % by weight of pigment and/or from 2 to 15 % by weight of matt-effect agent.

24. Composition according to any one of Claims 1 to 23, characterized in that it also contains adjuvants chosen from cosmetic or dermatological active agents, antioxidants, bactericides, melanin, moisturizing agents, sunscreens, fragrances, preserving agents and fillers.

25. Cosmetic and/or dermatological composition, characterized in that it consists of a composition as defined according to any one of Claims 1 to 21.

26. Use of a composition as defined according to any one of Claims 1 to 25 as a base for make-up products or for care products for the skin and/ or the face.

27. Use according to Claim 26, according to which the products are packaged in tubes or jars and are intended to be applied directly with the fingers onto the skin and/or the face.

## Patentansprüche

1. Zusammensetzung in Form eines wasserfreien Gels mit Fettphase, dadurch gekennzeichnet, daß sie in einer Fettphase, die kein Wachs enthält, mindestens enthält:
a) einen organomodifizierten Ton,
b) expandierte thermoplastische Hohlpartikel aus einem Polymer oder Copolymer, das aus einem ethylenisch ungesättigten Monomer oder einem Gemisch von ethylenisch ungesättigten Monomeren hergestellt ist, und
c) eine pyrogene Kieselsäure.

2. Zusammensetzung nach Anspruch 1, dadurch gekennzeichnet, daß die Fettphase in einem Anteil von 60 bis 85 Gew.-%, bezogen auf das Gesamtgewicht der Zusammensetzung, enthalten ist.

3. Zusammensetzung nach einem der Ansprüche 1 und 2, dadurch gekennzeichnet, daß die Fettphase mindestens ein Öl enthält, das unter pflanzlichen Ölen, Mineralölen, synthetischen Ölen, Siliconölen, phenylgruppenhaltigen oder hydroxygruppenhaltigen Silicongummis, fluorhaltigen Ölen und ihren Gemischen ausgewählt ist.

4. Zusammensetzung nach einem der Ansprüche 1 bis 3, dadurch gekennzeichnet, daß der organomodifizierte Ton das Produkt der Umsetzung eines Hectorits oder eines Montmorillonits mit einem quartären Ammoniumsalz ist.

5. Zusammensetzung nach Anspruch 4, dadurch gekennzeichnet, daß es sich bei dem quartären Ammoniumsalz um Stearyldimethylbenzylammoniumchlorid oder das quartäre Ammoniumchlorid der Formel 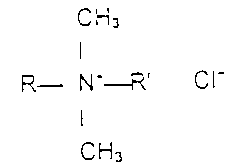 handelt, in der R und R' ein Gemisch von Alkylresten mit 14 bis 20 Kohlenstoffatomen darstellen, die von hydriertem Talg abgeleitet sind.

6. Zusammensetzung nach einem der Ansprüche 1 bis 5, dadurch gekennzeichnet, daß der organomodifizierte Ton in einer Konzentration von 0,5 bis 6 Gew.-% an Wirkstoff, bezogen auf das Gesamtgewicht der Zusammensetzung, enthalten ist.

7. Zusammensetzung nach einem der Ansprüche 1 bis 6, dadurch gekennzeichnet, daß der organomodifizierte Ton in Form eines Gels vorliegt, das aus dem Ton, einem pflanzlichen Öl, Mineralöl, tierischen Öl oder synthetischen Öl und einem Netzmittel besteht.

8. Zusammensetzung nach Anspruch 7, dadurch gekennzeichnet, daß der organomodifizierte Ton in Form eines Gels vorliegt, das aus dem Ton, einem Gemisch von Triglyceriden der Caprinsäure und der Caprylsäure und Propylencarbonat besteht.

9. Zusammensetzung nach einem der Ansprüche 1 bis 8, dadurch gekennzeichnet, daß die expandierten thermoplastischen Hohlpartikel Partikel aus einem expandierten Polymer oder Copolymer sind, das aus einem Monomer oder einem Gemisch von Monomeren hergestellt ist, die unter den Estern von Methacrylsäure oder Acrylsäure, Vinylidenchlorid, Acrylnitril, Styrol und seinen Derivaten, Butadien und seinen Derivaten ausgewählt sind.

10. Zusammensetzung nach Anspruch 9, dadurch gekennzeichnet, daß die thermoplastischen Hohlpartikel Partikel aus einem Polymer oder Copolymer von Methacrylat oder Methylmethacrylat, einem Styrol-Acrylnitril-Copolymer oder einem Copolymer aus Vinylidenchlorid und Acrylnitril oder Vinylchlorid sind.

11. Zusammensetzung nach einem der Ansprüche 9 und 10, dadurch gekennzeichnet, daß das Polymer oder Copolymer 0 bis 60 % Einheiten, die von Vinylidenchlorid abgeleitet sind, 20 bis 90 % Einheiten, die von Acrylnitril abgeleitet sind, und 0 bis 50 % Einheiten, die von einem Acrylmonomer oder einem Styrolmonomer abgeleitet sind, enthält, wobei die Summe der prozentualen Anteile 100 Gew.-% entspricht.

12. Zusammensetzung nach Anspruch 11, dadurch gekennzeichnet, daß die expandierten thermoplastischen Hohlpartikel Hohlpartikel aus einem expandierten Copolymer aus Vinylidenchlorid und Acrylnitril oder einem expandierten Terpolymer aus Vinylidenchlorid, Acrylnitril und Methylmethacrylat sind.

13. Zusammensetzung nach einem der Ansprüche 9 bis 12, dadurch gekennzeichnet, daß die Partikel eine Partikelgröße von 1 bis 100 µm aufweisen.

14. Zusammensetzung nach einem der Ansprüche 9 bis 13, dadurch gekennzeichnet, daß die thermoplastischen Hohlpartikel eine Dichte im Bereich von 15 bis 200 kg/m³ aufweisen.

15. Zusammensetzung nach einem der Ansprüche 1 bis 14, dadurch gekennzeichnet, daß die expandierten thermoplastischen Hohlpartikel in einer Konzentration von 0,1 bis 3 Gew.-%, bezogen auf das Gesamtgewicht der Zusammensetzung, enthalten sind.

16. Zusammensetzung nach einem der Ansprüche 1 bis 15, dadurch gekennzeichnet, daß die pyrogene Kieselsäure hydrophil oder hydrophob ist.

17. Zusammensetzung nach einem der Ansprüche 1 bis 16, dadurch gekennzeichnet, daß die pyrogene Kieselsäure in einer Konzentration von 0,1 bis 0,3 Gew.-%, bezogen auf das Gesamtgewicht der Zusammensetzung, enthalten ist.

18. Zusammensetzung nach einem der Ansprüche 1 bis 17, dadurch gekennzeichnet, daß sie außerdem ein Silicongel enthält, das besteht aus:
(A) einem teilweise vernetzten Polyorganosiloxan mit dreidimensionaler Struktur, das ausgewählt ist unter:
i) Polyorganopolysiloxanen, die Einheiten R₂SiO und RSiO_{1,5} und gegebenenfalls Einheiten R₃SiO_{0,5} und/oder SiO₂ enthalten, in denen die Reste R unabhängig voneinander Wasserstoff, Alkyl, Aryl, eine ungesättigte aliphatische Gruppe bedeuten, wobei das Gewichtsverhältnis der Einheiten R₂SiO zu den Einheiten RSiO_{1,5} im Bereich von 1/1 bis 30/1 liegt,
ii) in dem Siliconöl unlöslichen und quellbaren Polyorganopolysiloxanen, die durch Umsetzung eines Organowasserstoffpolysiloxans (1) mit einem Organopolysiloxan (2), das ungesättigte aliphatische Gruppen aufweist, hergestellt werden, wobei die Menge an Wasserstoff bzw. ungesättigten aliphatischen Gruppen in (1) bzw. (2) im Bereich von 1 bis 20 Mol-% liegt, wenn das Organopolysiloxan nicht cyclisch ist, und im Bereich von 1 bis 50 % liegt, wenn das Organopolysiloxan cyclisch ist;
(B) einem Siliconöl mit einer Viskosität kleiner als oder gleich 0,5 Pa·s; wobei das Gewichtverhältnis des teilweise vemetzten Polyorganosiloxans zu dem Siliconöl im Bereich von 5/95 bis 30/70 liegt.

19. Zusammensetzung nach Anspruch 18, dadurch gekennzeichnet, daß das Siliconöl unter geradkettigen Dimethylpolysiloxanen und Methylphenylpolysiloxanen mit niedrigem Polymerisationsgrad, Octamethylcyclopentasiloxan, Decamethyl-cyclopentasiloxan und Gemischen dieser beiden Verbindungen ausgewählt ist.

20. Zusammensetzung nach einem der Ansprüche 18 und 19, dadurch gekennzeichnet, daß das Silicongel aus einem geradkettigen Polydimethylsiloxan mit einer niedrigen Viskosität und einem teilweise vemetzten Polydimethylsiloxan mit dreidimensionaler Struktur besteht.

21. Zusammensetzung nach einem der Ansprüche 18 bis 20, dadurch gekennzeichnet, daß das teilweise vernetzte Polyorganosiloxan in einer Konzentration von bis zu 8 Gew.-% an Wirkstoff, bezogen auf das Gesamtgewicht der Zusammensetzung, enthalten ist.

22. Zusammensetzung nach einem der Ansprüche 1 bis 21, dadurch gekennzeichnet, daß sie außerdem mindestens ein Pigment und/oder mindestens ein Mattierungsmittel enthält.

23. Zusammensetzung nach Anspruch 22, dadurch gekennzeichnet, daß sie bis zu 20 Gew.-% Pigment und/oder 2 bis 15 Gew.-% Mattierungsmittel enthält.

24. Zusammensetzung nach einem der Ansprüche 1 bis 23, dadurch gekennzeichnet, daß sie außerdem Hilfsstoffe enthält, die unter kosmetischen oder dermatologischen Wirkstoffen, Antioxidantien, Bakteriziden, Melanin, Hydratisierungsmitteln, Sonnen-schutzfiltern, Parfüms, Konservierungsmitteln, Füllstoffen ausgewählt sind.

25. Kosmetische und/oder dermatologische Zusammensetzung, dadurch gekennzeichnet, daß sie aus einer wie in einem der Ansprüche 1 bis 21 definierten Zusammensetzung besteht.

26. Verwendung einer wie in einem der Ansprüche 1 bis 25 definierten Zusammensetzung als Grundlage für Schminkprodukte oder für Produkte zur Pflege der Haut und/oder des Gesichts.

27. Verwendung nach Anspruch 26, wobei die Produkte in einer Tube oder einem Tiegel verpackt sind und dafür vorgesehen sind, unmittelbar mit den Fingern auf die Haut und/oder das Gesicht aufgetragen zu werden.
